Europäisches Patentamt
European Patent Office
Office européen des brevets

(19)

(11) Publication number: **0 580 231 A1**

(12) **EUROPEAN PATENT APPLICATION**

(21) Application number: **93202090.2**

(22) Date of filing: **16.07.93**

(51) Int. Cl.[5]: **C07C 47/542**, C07C 45/56, C07C 22/04, C07C 17/14

(30) Priority: **23.07.92 IL 102624**
**14.06.93 IL 106008**

(43) Date of publication of application:
**26.01.94 Bulletin 94/04**

(84) Designated Contracting States:
**BE CH DE ES FR GB IT LI NL**

(71) Applicant: **IMI (TAMI) INSTITUTE FOR RESEARCH & DEVELOPMENT LTD.**
**Haifa-Bay P.O. Box 10140**
**IL-Haifa 26111(IL)**

(72) Inventor: **Zviely, Michael**
**3, Haim-Hazaz Street,**
**Savioney-Dania**
**Haifa(IL)**
Inventor: **Hanuka, Ezra**
**2, Haalon Street**
**Nesher(IL)**

(74) Representative: **Perani, Aurelio et al**
**c/o JACOBACCI-CASETTA & PERANI**
**Via Visconti di Modrone 7**
**I-20122 Milano (IT)**

(54) **Process for the preparation of 4-tert-butylbenzaldehyde.**

(57) In a process for the preparation of 4-*tert*-butylbenzaldehyde in high yield and purity a) 4-*tert*-butyltoluene is brominated using bromine and without a solvent, to obtain a mixture consisting essentially of 4-*tert*-butylbenzalbromide and 4-*tert*-butylbenzylbromide; and b) the mixture obtained in step (a) is reacted with water and a Sommelet reagent, optionally in the presence of a co-solvent, to obtain the desired product.

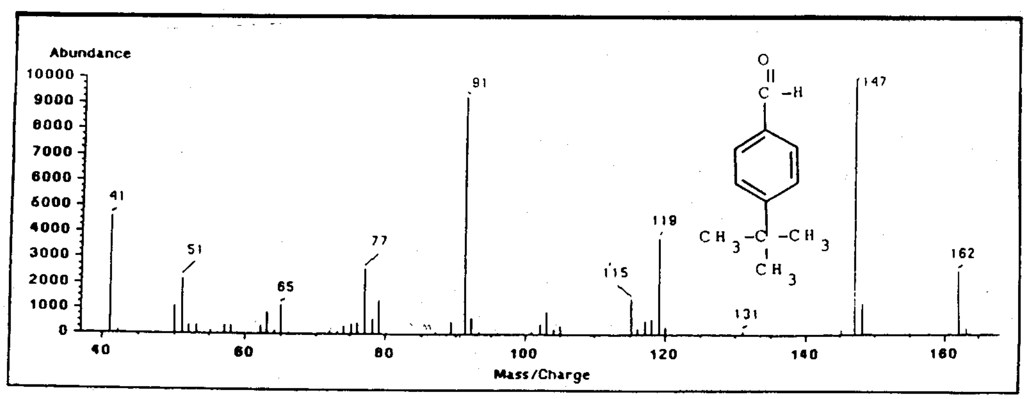


Fig. 4

EP 0 580 231 A1

## Field of The Invention

The present invention relates to the preparation of 4-*tert*-butylbenzaldehyde.

## Background of The Invention

4-*tert*-Butylbenzaldehyde (TBBZA) is used in the manufacturing of agricultural chemicals and fragrances, and is an intermediate for the "*Lily of the Valley*" fragrance, by the name of Lilial™ (4-*tert*–butyl-$\alpha$-methyldihydrocinnamaldehyde). Lilial™ is one of the most widely used ingredients in perfumes for soaps, detergents and cosmetics, with its floral, linden, lily-of-the-valley odor. It is equally important as an intermediate for the synthesis of the pesticide Fenpropimorph {4-[3-[4-(1,1-dimethylethyl)-phenyl]-2-methylpropyl-2,6(cis)-dimethylmorpholine}.

Lilial™ is prepared mainly by condensing TBBZA with propanal followed by catalytic hydrogenation. Fenpropimorph is prepared from Lilial™ by condensing it with 2,6-dimethylmorpholine followed by catalytic hydrogenation.

## The Prior Art

TBBZA has been prepared in the art mainly by the following processes:

- Halogenation of 4-*tert*-butyltoluene, followed by hydrolysis or Sommelet reaction;
- Oxidation of 4-*tert*-butyltoluene;
- Reduction of 4-*tert*-butylbenzoic acid.

German Patent No. 3.021.272 discloses a process in which 4-*tert*-butyltoluene is reacted with bromine, followed by hydrolysis with formic acid. The resulting product is obtained with a relatively not high yield (91.4%) and low purity (97.8%), after distillation, and still contains 2.2% TBT-Br.

U.S.S.R. Patent No. 1002284 teaches to react 4-*tert*-butyltbenzylchloride with HMTA in acetic acid. This reaction leads to low yields (55-75%) and purity (94.5%), and to the presence of 4-*tert*-butylbenzylacetate as an impurity which cannot be easily removed.

JP 60,248,640 teaches the preparation of benzaldehydes by hydrolyzing benzal halides over $Zn(OH)_2$ or $ZnCO_3$ catalysts.

## SUMMARY OF THE INVENTION

It has now been found, and this is an object of the present invention, that 4-*tert*-Butylbenzaldehyde (TBBZA) can be prepared by a convenient and economic process, using cheap raw materials, in 2 steps:

1) Radical bromination using bromine, without solvent, on 4-*tert*-butyltoluene to form 4-*tert*-butylbenzylbromide (TBT-Br) and 4-*tert*-butylbenzalbromide (TBT-$Br_2$) mixture; and

2) Hydrolysis of the TBT-$Br_2$/TBT-Br mixture - by addition of water, to form TBBZA, in the presence of a Sommelet reagent.

As will be appreciated by the skilled chemist, the process of the invention permits to utilize TBT-Br and to remove it from the resulting product at the same time, in contrast to prior art processes in which TBT-Br is not reacted.

It has further been found, and this is another object of the invention, that operating in the absence of co-solvents, which is very convenient from the processual point of view, permits to obtain a highly pure product.

It has also been found, and this is a further object of the invention, that it is desirable to operate in the bromination reaction with $Br_2$:TBT ratios not exceeding 1.97:1. This is contrary, e.g., to the teachings of the aforementioned German Patent No. 3.021.272, which teaches to employ ratios of 2.16:1.

Furthermore, the process comprises adjusting the pH of the reaction mixture, which is very low after the bromination step, to a value between 2 and 7, in order to remove HBr from the solventless reaction mixture, which otherwise inhibits the reaction of the Sommelet reagent (i.e., HMTA).

Thus the process for the preparation of 4-*tert*-butylbenzaldehyde according to the invention comprises the steps of:

a) brominating 4-*tert*-butyltoluene using bromine and without a solvent, to obtain a mixture consisting essentially of 4-*tert*-butylbenzalbromide and 4-*tert*-butylbenzylbromide;

b) reacting the mixture obtained in step (a) with water in the presence of a small amount of a Sommelet reagent, to obtain the desired product.

By "Sommelet Reagent" is meant to indicate a reagent which reacts in the so-called Sommelet reaction, e.g., HMTA or mixtures of formaldehyde-ammonia, as more fully described in Scheme A below. HMTA is the preferred Sommelet reagent, and therefore reference will be made to it in most cases below, it being understood that any other suitable Sommelet reagent can be employed in its stead, as appropriate. The amount of Sommelet reagent depends of course from the amount of TBT-Br present in the mixture, and sufficient reagent must be provided, the amount of which will be apparent to the routineer.

As stated, although not absolutely necessary, it is advantageous and economic to carry out the two reaction steps consecutively in the same vessel. Separation of the reaction steps, however, although not advantageous, does not exceed the scope of the invention.

The preferred temperature range in which the bromination reaction is carried out is 145° - 220°C, preferably 183° - 190°C. The reaction can also be carried out at a lower temperature, while retaining many of the advantages of the invention. This, however, is not preferred, because at temperatures below 145°C the addition of light is required.

The preferred range for carrying out the Sommelet reaction is 90°-105°C, preferably at or near reflux. At temperatures below 90°C the hydrolysis is relalively slow, and therefore it will normally be desirable to operate above this temperature, although lower temperatures are possible. If it is desired to perform the reaction at temperatures above 105°C, this requires pressure since the water solution boils, and therefore operating at such high temperatures is also generally avoided.

According to a preferred embodiment of the invention, the bromination reaction is carried out in the substantial absence of light. However, although not preferred in view of the technological complications involved, the reaction with light is of course also possible. According to another preferred embodiment of the invention the pH of the reaction mixture obtained in reaction step (a) is adjusted to be in the range 2 to 7, preferably 4 - 5, before reaction step (b) is carried out, by adding a suitable base.

As stated, it is preferred to carry out the hydrolysis reaction without a co-solvent, since this streamlines the process and renders it simpler and more economic. However, the use of a co-solvent (e.g., carboxylic acids, such as acetic acid, or alcohols, such as ethanol, etc.) does not exceed the scope of the invention, as many of the advantages of the process of the invention are retained even if a co-solvent is employed.

Still another object of the invention is to provide a process wherein the TBT-CN impurity found in the reaction mixture is destroyed before work-up of the product begins. This step is very convenient because the TBT-CN impurity is very difficult to remove by normal work-up procedures, and prior art processes may lead to products containing such impurity. According to a preferred embodiment of the invention the TBT-CN impurity is hydrolyzed in the crude mixture, before distillation begins. Preferably, the hydrolysis is effected under acidic conditions, by adding, e.g., acids such as $H_2SO_4$, $H_3PO_4$, and the like. Hydrolysis can also be effected under basic conditions, by adding a suitable base, e.g., NaOH or KOH. Hydrolysis under basic conditions is not preferred, however, as will be apparent to the skilled chemist.

It has further been found, and this is another object of the invention, that it is advantageous to carry out a first evaporation step, before hydrolysis of the TBT-CN impurity is undertaken, and that doing so increases the final yield by 5-10%, while high molecular weight by-products remain in the evaporation residue. These high molecular weight by-products, when operating according to the process described in the parent application, decompose during distillation increasing the impurity content of the final product. Thus, carrying out this preliminary evaporation is normally preferred.

The invention permits to prepare 4-t-butylbenzaldehyde in high yield and purity. Bromination is carried out in a yield of about 96%, the overall yield of the process, after purification, being about 80%. Purity obtained is about 99.5%.

Bromine can be added in any suitable manner, e.g., in liquid or gaseous form. The results obtained with gaseous bromine are comparable to those obtained with liquid bromine.

All the above and other characteristics and advantages of the invention will be better understood through the following detailed description of preferred embodiments.

## Detailed Description

The overall process is shown in Scheme A:

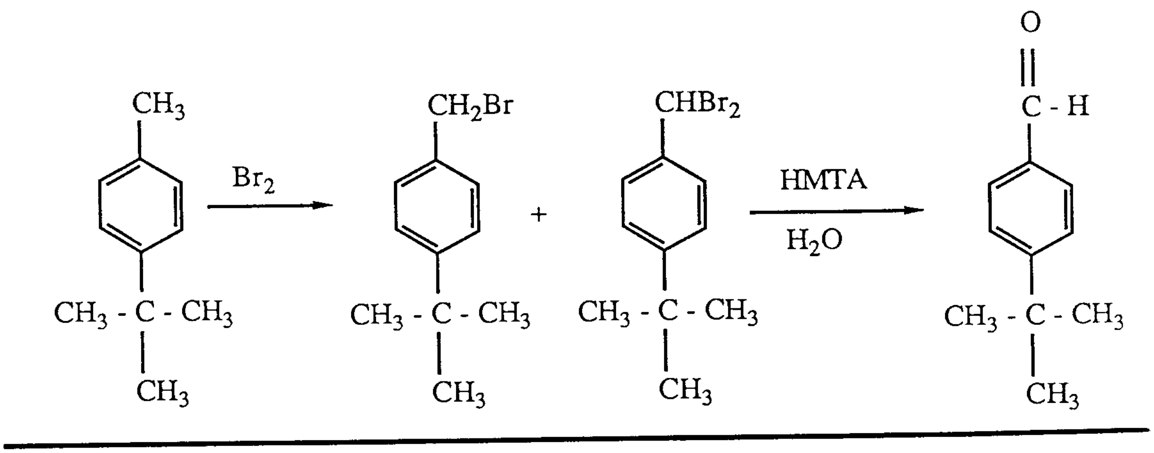


SCHEME A

## List Of Abbreviations

In the following description, the abbreviations listed below will be used, for the sake of brevity:

| | |
|---|---|
| TBBZA | - *tert*-butylbenzaldehyde |
| HMTA | - hexamethylenetetraamine |
| TBT | - *tert*-butyltoluene |
| pTBT | - 4-*tert*-butyltoluene |
| mTBT | - 3-*tert*-butyltoluene |
| TBT-Br | - *tert*-butylbenzylbromide |
| $TBT\text{-}Br_2$ | - *tert*-butylbenzalbromide |
| $TBT\text{-}Br_3$ | - *tert*-butylbenzotribromide |
| Br-TBT | - bromo-*tert*-butyltoluene |
| TBT-Cl | - *tert*-butylbenzylchloride |
| TBT-COOH | - *tert*-butylbenzoic acid |
| TBT-CN | - *tert*-butylbenzonitrile |
| $TBT\text{-}NMe_2$ | - N,N-dimethyl-*tert*-butylbenzylamine |
| DBDMH | - N,N'-dibromo-5,5-dimethylhydantoin |
| DMH | - 5,5-dimethylhydantoin |
| AIBN | - azoisobutyronitrile |
| DiBr | - TBT derivatives which contain 2 bromine atoms, either in the benzylic position or on the ring |
| TriBr | - TBT derivatives which contain 3 bromine atoms, either in the benzylic position or on the ring |

## Brief Description of the Drawings

- Fig. 1 is the GC spectrum of the brominated TBT of Example 1;
- Fig. 2 is the $^1$H-NMR spectrum of the product of Example 1;
- Fig. 3 is the GC spectrum of the crude Sommelet reaction product of Example 2;
- Fig. 4 is the mass-spectrum of TBBZA;

TBT can be brominated using other bromine carriers, e.g., dibromodimethylhydantoin (DBDMH) or N-bromosuccinimide (NBS), and at different temperatures. Also solvents can be used, such as $CCl_4$, 1,2-dichloroethane, dibromomethane, 1,1,2,2-tetrachloroethane,tetrachloroethylene, ethylenedibromide, chlorobenzene, etc.

**Analytical Procedures**

GC Analyses were conducted on a HEWLETT-PACKARD 5890 Series II Gas Chromatograph, using a 15 m capillary column (ID = 0.25 mm) packed with crosslinked dimethyl-silicone. The temperature was programmed: T1 = 90°C; dt/dT = 15°C/min.; T2 = 300°C.

GCMS Analyses were performed on a HEWLETT-PACKARD 5890 Gas Chromatograph equipped with a 5970 series Mass Selective detector, using 12.5 m capillary column (ID = 0.25mm) packed with crosslinked dimethylsilicone. The temperature was programmed: T1 = 70°C; dT/dt = 15°C/min.; T2 = 270°C.

$^1$H-NMR Analyses were conducted on a 200 MHz BRUKER WP 200 SY spectrometer for ~5% solutions in deuteriochloroform or in $d_6$-acetone. Calibrated $^1$H-NMR analyses were performed using 1,1,2,2-tetrachloro-ethane (δ 5.9 ppm) as standard.

Aldehyde contents was analyzed by oximation method, which is based on the reaction between TBBZA and hydroxylamine hydrochloride and titrating the HCl evolved in the reaction with NaOH solution. In this method the product concentration is analyzed after reaction with hydroxylamine hydrochloride, according to the following equation:

RCHO + $NH_2OH$•HCl --------> RCH=NOH + $H_2O$ + HCl

The quantity of evolved HCl is stoichiometric to the aldehyde quantity, and therefore the acid is titrated by a base with accurate concentration.

**Test Procedure**

(1) Weigh accurately 1.9 - 2.3 gr sample into a beaker;
(2) Add 50 ml hydroxylamine hydrochloride, cover the beaker and stir for about 20 minutes;
(3) Titrate with 1N NaOH (factorized).

**Example 1**

**Solventless Bromination Using Bromine**

Into a 4-necked round bottom flask (3 L), equipped with mechanical stirrer, condenser, a thermocouple and a water trap for the HBr evolving, was introduced:

| | |
|---|---|
| 4-*tert*-butyltoluene | 296.5 gr (345 ml, 2 mole) |

The TBT was heated to 170°C with vigorous stirring, and then was added from above, during ~7 hrs.:

| | |
|---|---|
| bromine | 632.8 gr (204 ml, 3.96 mole) |

After the addition was completed, the reaction was heated for additional 20 min. The crude brown oily product which solidifies at room temperature weighed 590.62 gr.
The product mixture was analyzed by GC (non-calibrated) to give the following results:

| | |
|---|---|
| *tert*-butylbenzylbromide | 7.16% |
| *tert*-butylbenzalbromide | 86.6% |
| dibromo derivatives | 1.15% |
| tribromo derivatives | 2.7% |
| *tert*-butylbenzoic acid | 2.03% |

Calibrated $^1$H-NMR analysis showed the following results:

| | |
|---|---|
| *tert*-butylbenzylbromide<br>*tert*-butylbenzalbromide | 5.36% ( 31.65 gr, 0.139 mole)<br>94.1% (555.8 gr, 1.81 mole) |

The yield of the reaction is 97%.

**Example 2**

**Sommelet Reaction of Bromination Product**

The product mixture from Example 1 was cooled to 45-50°C, and into the reactor which contained already:

| | |
|---|---|
| TBT-Br/TBT-$Br_2$ (5.36%:94.1%) | 583.3 gr |

were introduced at this temperature:

| | |
|---|---|
| $H_2O$<br>NaOH<br>HMTA | 1.2 L<br>2.2 gr (0.055 mole) (until pH 4-5), and<br>78 gr (0.56 mole) |

The solution was stirred and heated to reflux temperature (~105°C) for 4 hrs.

The mixture was cooled to ambient temperature and the phases were separated. The upper organic (brown) layer contained the following results (GC):

- 2.11% mTBBZA,
- 90.8% pTBBZA,
- 0.49% TBT-CN,
- 1.00% TBT-COOH,
- 0.14% TBT-Br,
- 0.57% bromo-*tert*-butylbenzaldehyde
- ~1% di-bromo derivatives

The organic layer weighed 318.2 gr. Yield of TBBZA was 94%. The organic layer was taken to fractional distillation.

**Example 3**

**Distillation of *tert*-Butylbenzaldehyde**

TBBZA was purified by fractional distillation *in vacuo*. The purity of TBBZA obtained was >99.5% (meta and para isomers).

Crude TBBZA which was obtained after Sommelet reaction of the benzyl bromides, was put to fractional distillation. Before the distillation began, conc. sulfuric acid (3.5% relative to the organic phase) was added in order to hydrolyze the TBT-CN impurity, which is not possible to separate by distillation.

**Example 4**

**Bromination of TBT with Higher Bromine/TBT Molar Ratio**

Into a 4-necked round bottom flask (0.5 L) equipped with mechanical stirrer, condenser, a thermocouple and a water trap for the HBr evolving, was introduced:

| | |
|---|---|
| 4-*tert*-butyltoluene | 120.0 gr (0.785 mole) |

The TBT was heated to 165-170°C with vigorous stirring, and then there was added to the above during ~5.5 hours:

| | |
|---|---|
| bromine | 272.0 gr (88 ml, 1.7 mole) |

After the addition was completed, the reaction was heated for an additional 30 minutes. The crude brown oily product weighed 245.1 gr. The product mixture was analyzed by GC (non-calibrated) to give the following results:

| | |
|---|---|
| *tert*-butylbenzylbromide | 0.84% |
| *tert*-butylbenzalbromide | 80.0% |
| dibromo derivatives | 1.0% |
| tribromo derivatives | 14.5% |
| *tert*-butylbenzoic acid | 1.6% |

**<u>Example 5 (Comparative)</u>**

**<u>Bromination of TBT Using Bromine in Chlorobenzene as Solvent</u>**

Into a 4-necked round bottom flask (1 L) equipped with mechanical stirrer, condenser, a thermocouple, a water trap for the HBr evolving and a 160 W lamp were introduced:

| | |
|---|---|
| 4-*tert*-butyltoluene | 148.25 gr (172.4 ml, 1 mole) |
| chlorobenzene | 330 gr (300 ml) |

The mixture was refluxed at 140-146 °C with vigorous stirring, and then there was added dropwise to the above during 3.5 hours:

| | |
|---|---|
| bromine | 279.0 gr (90 ml, 1.74 mole) |

After the addition was completed, the reaction was refluxed for an additional 30 minutes, and the chlorobenzene was evaporated *in vacuo* (bath temperature ~90 °C, ~35 mmHg). A faint red color of bromine was seen with the solvent. The remaining crude product was brown oil. The product mixture `was analyzed by GC (non-calibrated) to give the following results:

| | |
|---|---|
| *tert*-butylbenzylbromide | 22.6% |
| *tert*-butylbenzalbromide | 73.0% |
| *tert*-butylbenzotribromide | 1.8% |
| chlorobromobenzenes | 0.3% |
| *tert*-butylbenzoic acid | 0.3% |

Calibrated $^{1}$H-NMR analysis showed the following results:

| | |
|---|---|
| *tert*-butylbenzylbromide | 13.6% (42.3 gr, 0.187 mole) |
| *tert*-butylbenzalbromide | 70.6% (219.63 gr, 0.715 mole) |

The yield of the reaction was 90%.

**<u>Example 6 (Comparative)</u>**

**<u>Bromination of TBT with DBDMH/AIBN/Chlorobenzene System</u>**

Into a 3-necked round bottom flask (3 L) equipped with mechanical stirrer, condenser and a thermocouple were introduced:

| | |
|---|---|
| *tert*-butyltoluene<br>chlorobenzene | 100 gr (0.67 mole)<br>1 L |

The mixture was refluxed at 132 °C with vigorous stirring, and then there was added continuously a slurry of:

| | |
|---|---|
| DBDMH<br>AIBN<br>chlorobenzene | 165.1 gr (0.58 mole)<br>6.6 gr (0.039 mole)<br>500 ml |

After the addition was completed, the reaction was allowed to cool to room temperature, the DMH was filtered and the products mixture solution was analyzed by GC to give the following results:

| | |
|---|---|
| *tert*-butylbenzylbromide<br>*tert*-butylbenzalbromide<br>*tert*-butylbenzotribromide | 33.8%<br>58.9%<br>3.3% |

## Example 7

### Sommelet-Hydrolysis 2-Stage Reaction on Benzylbromides Mixture with Solvent

Into a 5-liter flask equipped with condenser, nitrogen bubbler, mechanical stirrer and a thermocouple were introduced at room temperature:

| | |
|---|---|
| TBT-Br/TBT-$Br_2$ (13.6%:70.6%)<br>chlorobenzene<br>HMTA | 622 gr<br>770 gr (700 ml, 6.84 mole)<br>177 gr (1.26 mole) |

The solution was stirred and heated to ~40 °C, and the reaction temperature climbed to 88 °C. At ~60 °C, a white hexaminium salt was formed. The temperatures were 88 > 65 °C (the set point was on 65 °C, but it was lowered slowly from 88 to 65 °C). After 40 minutes, a non-calibrated GC analysis showed the following results:

| | |
|---|---|
| chlorobromobenzene<br>HMTA<br>TBBZA<br>TBT-Br<br>TBT-COOH<br>TBT-$Br_2$<br>TBT-$Br_3$ | 0.4%<br>2.5%<br>1%<br>0.56%<br>0.9%<br>90.4%<br>2.25% |

After twenty more minutes, 2 liters of water were added, and the mixture was refluxed at 92 °C, pH = 3 for 105 minutes. Then, 750 ml of HBr (aqueous pH = 0, from bromination step) were added at reflux, and the mixture was refluxed at 92 °C, pH = 1.5 for ~9 more hours.

The mixture was cooled to ~80 °C and the phases were separated. The upper organic (brown) layer was evaporated in *vacuo* (35 mmHg, 90 °C). Non-calibrated GC analysis showed the following results:

| | |
|---|---|
| chlorobromobenzenes | 0.4% |
| TBBZA | 92.7% |
| TBT-CN | 1.21% |
| TBT-$NMe_2$ | 0.9% |
| TBT-COOH | 1% |

The organic layer weighed 350.2 gr. The yield ofTBBZA (relative to TBT-Br/TBT-$Br_2$) was 86.7% (by GC calibrated).

**Example 8**

**Effect of pH Adjustment**

After the bromination stage, the product mixture was cooled to 65°C and into a 4-necked (5 L) reactor equipped with condenser, nitrogen bubbler, mechanical stirrer and a thermocouple which contained already:

| | |
|---|---|
| TBT-Br/TBT-$Br_2$ (20.45%, 71.5%) | 290.3 gr |

there was added:

| | |
|---|---|
| chlorobenzene | 330 gr |

The solution was stirred at 65°C, and there was added:

| | |
|---|---|
| HMTA | 87 gr |

The reaction mixture was heated to 90°C. No salt formation was observed. After 60 minutes, a non-calibrated GC analysis showed the following results:

| | |
|---|---|
| TBT-Br | 14.2% |
| TBT-$Br_2$ | 79.0% |

After 30 more minutes, a non-calibrated GC analysis showed the following results:

| | |
|---|---|
| TBT-Br | 12.7% |
| TBT-$Br_2$ | 85.0% |

At this stage, sodium hydroxide was added at 80°C until pH reached ~6, and salt formation was observed. No TBT-Br was observed by GC.

Then, water (1.25 L) was added, and the mixture refluxed at 97°C. The mixture was cooled to ~80°C, and the phases were separated. The upper organic (brown) layer was evaporated i*n vacuo* (35 mmHg, 90°C). Non-calibrated GC analysis showed the following results:

| | |
|---|---|
| TBBZA | 87% |
| TBT-CN | 0.2% |
| other compounds | 12.8% |

## Example 9

### Bromination of TBT at 183°-190°C

Into a 4-necked round bottom flask (0.5 L), equipped with mechanical stirrer, condenser, a thermocouple and a water trap for the HBr evolving, was introduced:

| | |
|---|---|
| 4-*tert*-butyltoluene | 120.0 gr (0.785 mole) |

The TBT was heated to 183°-190°C with vigorous stirring, and then was added from above, during ~3.5 hrs.:

| | |
|---|---|
| bromine | 247.4 gr (79 ml, 1.54 mole) |

After the addition was completed, the reaction was heated for additional 30 min. The crude brown oily product which solidifies at room temperature weighed 237.4 gr. The product mixture was analyzed by GC (non-calibrated) to give the following results:

| | |
|---|---|
| *tert*-butylbenzylbromide | 5.1% |
| *tert*-butylbenzalbromide | 89.2% |
| dibromo derivatives | 1.1% |
| tribromo derivatives | 4.4% |

The experiment was repeated a number of times, as detailed in Table I below:

### Table I

| $Br_2$/TBT mol.rat. | MODE Total | TIME hr | TEMP °C | TBT-Br % | TBT-$Br_2$ % | DiBr % | TriBr % | Good* Br% | GC% |
|---|---|---|---|---|---|---|---|---|---|
| 1.84 | gas | 1.25 | 175 | 7.3 | 90.6 | 0.26 | 1.54 | 97.9 | 99.7 |
| 1.99 | liq. | 8.5 | 170 | 4.5 | 89 | 1.43 | 4.04 | 93.5 | 98.97 |
| 1.99 | liq. | 3.5 | 186 | 5.47 | 88.45 | 1.24 | 4.42 | 93.92 | 99.58 |
| 1.99 | gas | 3.5 | 192 | 8.69 | 89.4 | 0.25 | 1.58 | 98.09 | 99.92 |
| 1.99 | liq. | 3.5 | 195 | 5.07 | 91.65 | 0.27 | 2.22 | 96.72 | 99.21 |
| 1.99 | liq. | 1.45 | 195 | 5.92 | 89.82 | 0.8 | 3.2 | 95.74 | 99.74 |
| 2.02 | liq. | 5 | 140 | 5.8 | 87.6 | 2.1 | 2.9 | 93.4 | 98.4 |

* Good Br = The compounds TBT-$Br_2$ and TBT-Br

## Example 10

### Destruction of TBT-CN with pre-distillation

The 4-t-butylbenzonitrile which is obtained as impurity after the Sommelet reaction, and which cannot be separated from the product by distillation, is hydrolyzed to give the benzoic acid derivative. The hydrolysis was carried out both before the evaporation stage and after the evaporation stage.

Into a reaction flask there was introduced crude material (290 gr) containing:

| | |
|---|---|
| TBBZA: | 98.3% |
| TBT-CN: | 0.5% |

Then concentrated $H_2SO_4$ (98%, 7.4 gr) was also added, and the mixture was stirred at 150 °C for 30 minutes. The crude product obtained weighed 295 gr and showed the following results in GC:

| | |
|---|---|
| TBBZA: | 97.3% |
| TBT-CN: | non detectable. |

This procedure was repeated with and without preliminary evaporation, and the results are detailed in Table II below. From these results it can be seen that evaporating the crude product before hydrolysis considerably reduces the operation time.

## Table II

| Time min | TBBZA % | TBT-CN % | TEMP. °C | $H_2SO_4$ / TBT-CN (w/w) | $H_2SO_4$ / Crude (w/w) | PRODUCT |
|---|---|---|---|---|---|---|
| 0 | 97.5 | 1.2 | | | | After |
| Evaporation | | | | | | |
| 10 | 95.4 | 0 | 177 | 4 | 5.20% | |
| | 93.9 | 0.5 | | | | Crude |
| 0 | 98.3 | 0.56 | 150 | 4.02 | 2.41% | After |
| Evaporation | | | | | | |
| 30 | 97.3 | 0 | | | | |
| 0 | 90.4 | 0.4 | | | | Crude |
| 10 | 90 | 0.096 | 150 | 8 | 3.43% | Without Prior |
| 25 | 88.4 | 0.067 | | | | evaporation |
| 60 | 85.4 | 0.07 | | | | |
| 120 | 85 | 0 | | | | |

The above description and examples have been given for the purpose of illustration and are not intended to be limitative. Many variations can be made in the invention. For instance, different temperatures can be employed, and different bromine carriers can be used, or different reagents can be used instead of HMTA (e.g., a mixture of formaldehyde-ammonia) as apparent to the skilled chemist, all without exceeding the spirit of the invention.

### Claims

**1.** A process for the preparation of 4-*tert*-butylbenzaldehyde in high yield and purity, comprising the steps of:

a) brominating 4-*tert*-butyltoluene using bromine and without a solvent, to obtain a mixture consisting essentially of 4-*tert*-butylbenzalbromide and 4-*tert*-butylbenzylbromide;

b) reacting the mixture obtained in step (a) with water and a Sommelet reagent, optionally in the presence of a co-solvent, to obtain the desired product.

**2.** A process according to claim 1, wherein the two steps are carried out consecutively in the same vessel.

**3.** A process according to claim 1 or 2, wherein the Sommelet reagent is hexamethylenetetraamine.

**4.** A process according to any one of claims 1 to 3, wherein the bromination reaction is carried out at a temperature in the range 145°-220°C.

**5.** A process according to claim 1, wherein the bromination reaction is carried out in the temperature range of 183°-190°C.

**6.** A process according to any one of claims 1 to 5, wherein the Sommelet reaction is carried out in the temperature range of 90°-105°C, preferably at or near reflux.

**7.** A process according to claim 1, wherein the bromination reaction is carried out in the substantial absence of light.

**8.** A process according to claim 1, wherein the bromination reaction is carried out in the presence of light.

**9.** A process according to claim 1, wherein the pH of the reaction mixture obtained in step (a) is adjusted to be in the range 2 to 7, preferably 4 - 5, before step (b) is carried out.

**10.** A process according to claim 9, wherein the pH is adjusted by adding a suitable base.

**11.** A process according to any one of claims 1 to 10, wherein the TBT-CN impurity found in the reaction mixture is destroyed by hydrolyzing it.

**12.** A process according to claim 11, wherein pre-evaporation of the reaction mixture is carried out before destroying the TBT-CN impurity found in the reaction mixture by hydrolysis.

**13.** A process according to claim 11 or 12, wherein the hydrolysis is effected by adding an acid.

**14.** A process according to claim 13, wherein the acid is sulfuric acid.

**15.** A process according to claim 1, wherein the co-solvent is selected from carboxylic acids, preferably acetic acid and an alcohol, preferably ethanol.

**16.** A process according to claim 1, wherein bromination is carried out using gaseous bromine.

**17.** A process according to claim 1, wherein bromination is carried out using liquid bromine.

**18.** A process for the preparation of 4-*tert*-butylbenzaldehyde, essentially as described and illustrated.

**19.** 4-*Tert*-butylbenzaldehyde, whenever prepared by the process of any one of claims 1 to 18.

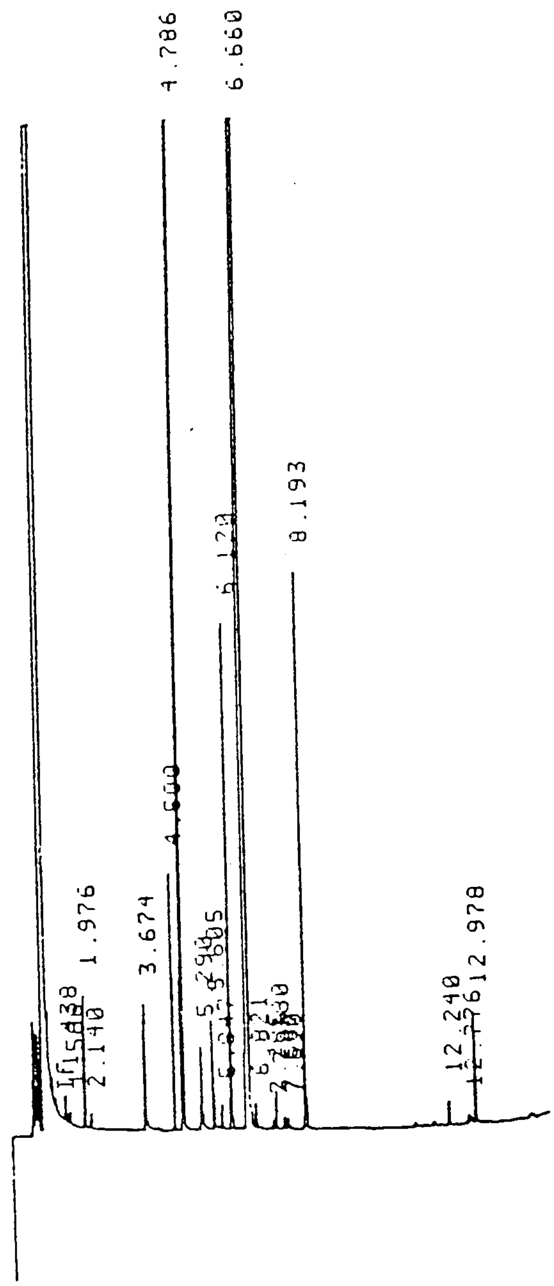


Fig. 1

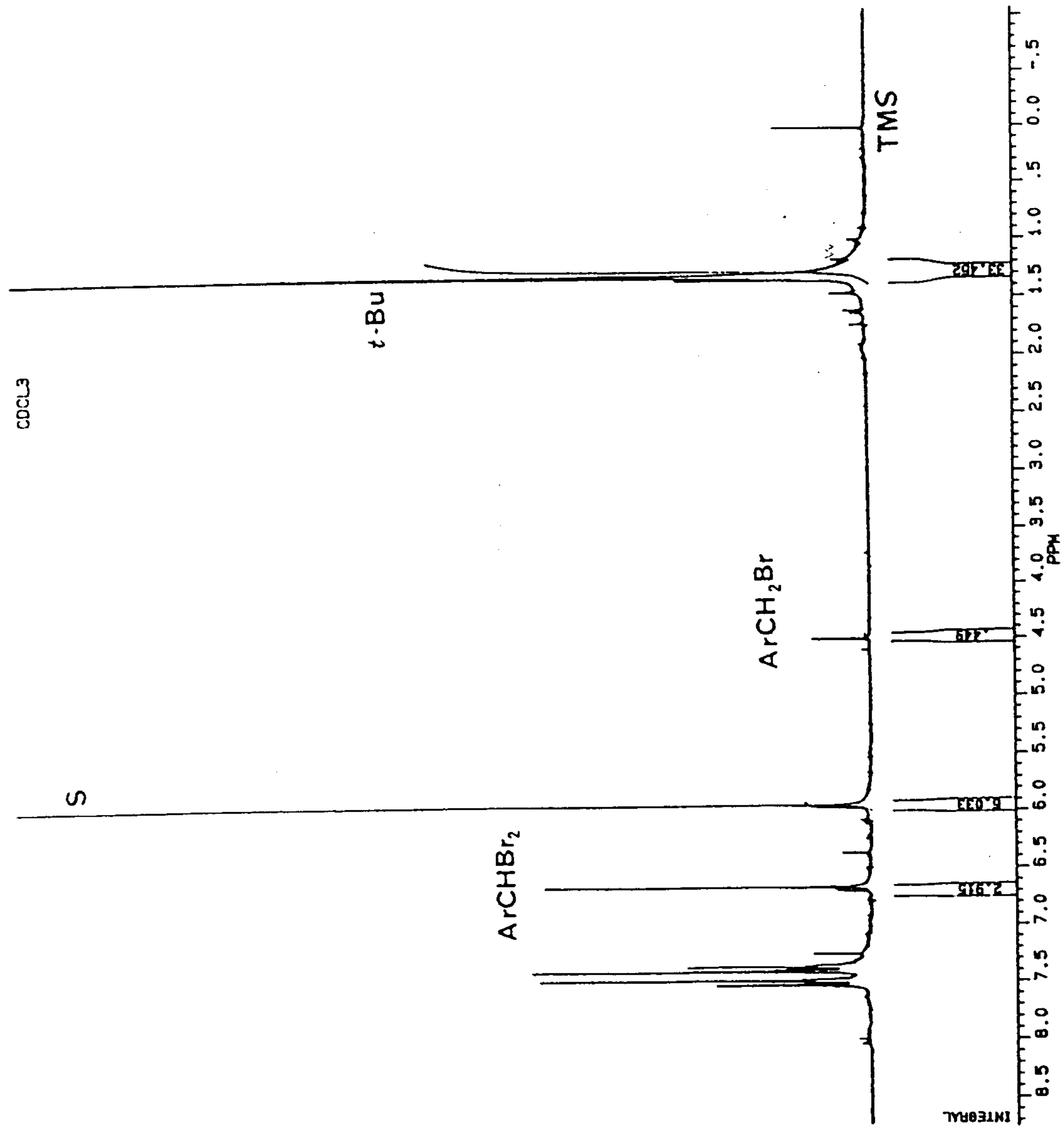
CDCL3
t-Bu
TMS
ArCH2Br
S
ArCHBr2
PPM
INTEGRAL
33.462
.449
6.033
2.915

Fig. 2

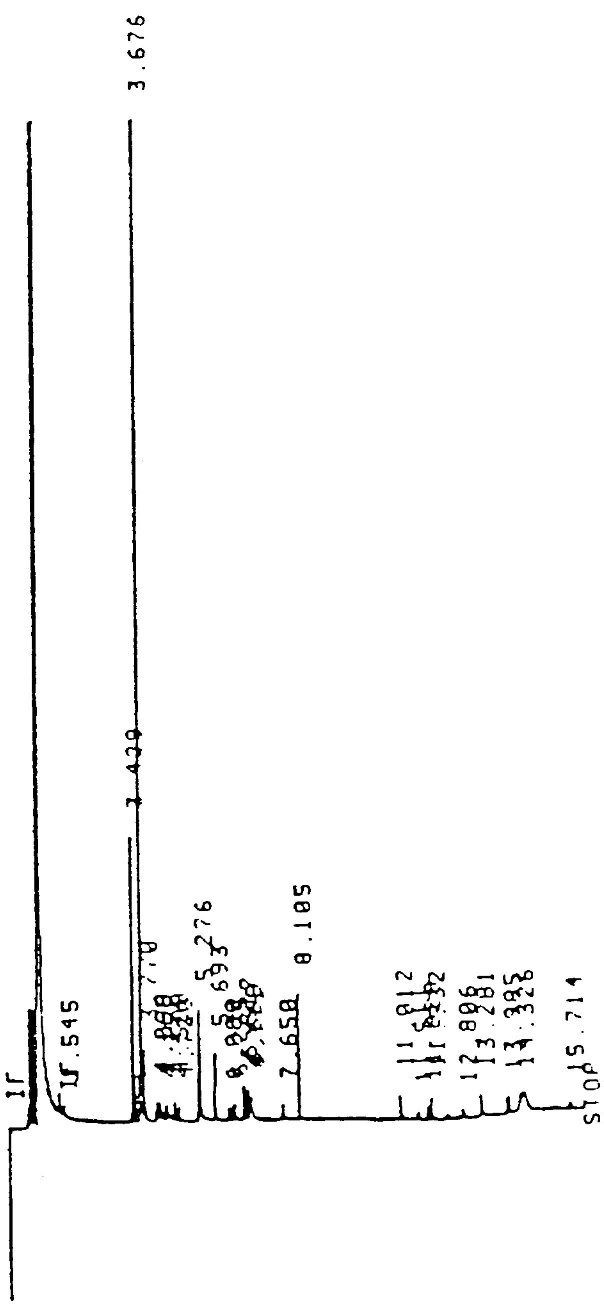


Fig. 3

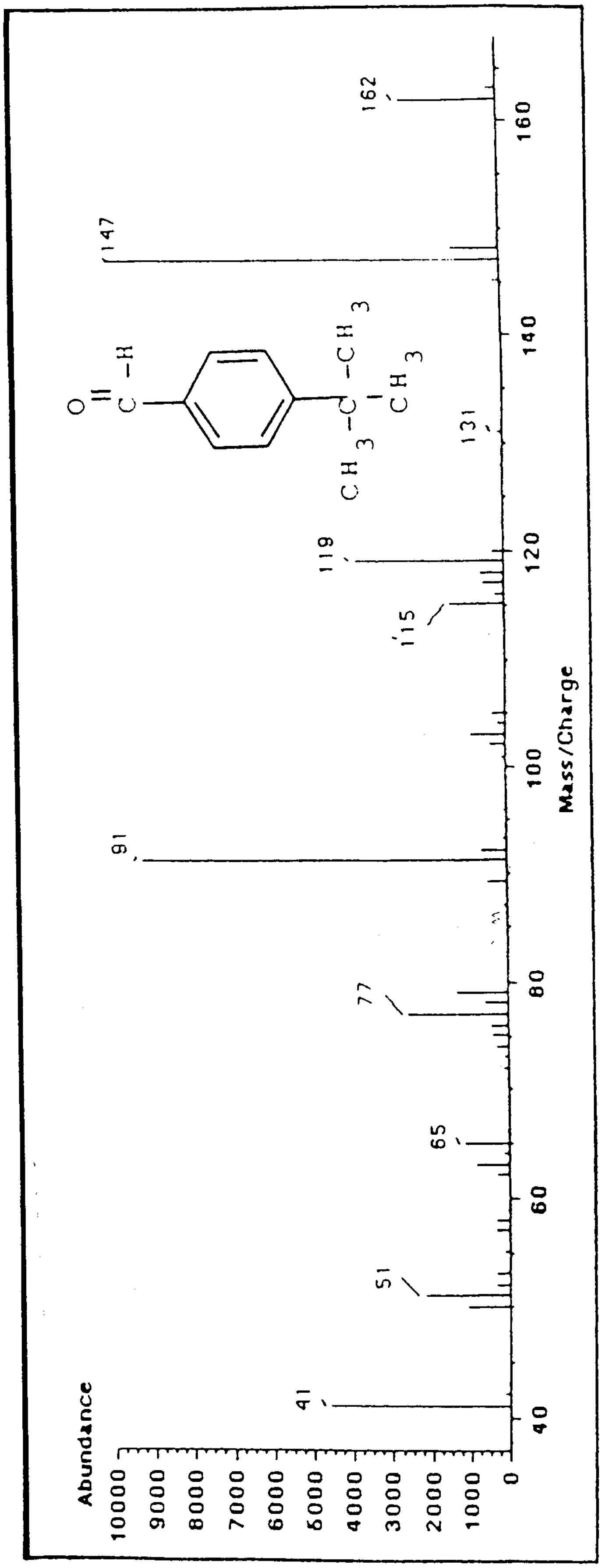

Abundance
10000
9000
8000
7000
6000
5000
4000
3000
2000
1000
0
40
60
80
100
120
140
160
Mass/Charge
41
51
65
77
91
115
119
131
147
162


Fig. 4

European Patent Office

# EUROPEAN SEARCH REPORT

Application Number
EP 93 20 2090

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | EP-A-0 028 725 (BASF AKTIENGESELLSCHAFT)<br>* the whole document *<br>--- | 1 | C07C47/542<br>C07C45/56<br>C07C22/04<br>C07C17/14 |
| Y | FR-A-2 528 034 (L. GIVAUDAN & CIE S.A.)<br>* the whole document *<br>--- | 1 | |
| Y | DE-A-30 21 728 (BAYER AKTIENGESELLSCHAFT)<br>* the whole document *<br>----- | 1 | |
| | | | TECHNICAL FIELDS SEARCHED (Int.Cl.5) |
| | | | C07C |

The present search report has been drawn up for all claims

| Place of search | Date of completion of the search | Examiner |
|---|---|---|
| THE HAGUE | 28 October 1993 | BONNEVALLE, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document



EPO FORM 1503 03.82 (P04C01)